# EUROPEAN PATENT APPLICATION

(11) **EP 3 090 997 A1**
(43) Date of publication of application: **09.11.2016**
(21) Application number: 15166246.7
(22) Date of filing: 04.05.2015
(51) Int. Cl.: C07C 29/48, C07C 31/04

(54) **A PROCESS FOR METHANE TO METHANOL CONVERSION AT LOW TEMPERATURE**

(71) Applicant: Paul Scherrer Institut, 5232 Villigen (CH)
(72) Inventor: Ranocchiari, Marco, 4310 Rheinfelden (CH); Tomkins, Patrick, 41812 Erkelenz (DE); Van Bokhoven, Jeroen A., 8044 Zürich (CH)
(74) Representative: Fischer, Michael

(57) **Abstract**

It is the objective of the present invention to provide a process for the conversion of methane into methanol that has a high selectivity, while providing advantageous and energy-saving conditions for this process.

This objective is achieved according to the present invention by a process for low temperature and isothermal methane to methanol conversion at temperatures below 280°C using oxygen; comprising the steps of:
a) activating a catalyst material with an oxygen-containing gas, preferably molecular oxygen, or an inert gas such as helium, nitrogen and argon;
b) feeding methane to the activated catalyst material thereby reacting the methane to a methanol precursor;
c) reacting and/or desorbing the methanol precursor from the catalyst material by treating with a gas or a liquid such as water, protic molecules, and/or CO;

wherein the steps mentioned above are executed in successive order, thereby applying a temperature in the range of 20 to 280°C, providing methane and the oxygen-containing gas at ambient pressure or at a pressure up to 250 bar, and using as catalyst material a transition or multi-metal loaded porous material.

This process allows the safe and selective conversion of methane to methanol at high methanol yields and advantageous energy consumption. Major improvement in the present process is the use of low temperature, possibly in isothermal conditions and in all cases, significantly below the temperatures so far disclosed in the prior art.

## Description

The present invention relates to a process for methane to methanol conversion at low temperature.

The direct conversion of methane to methanol has always been a major challenge, but solving this problem is very rewarding, as nowadays a major part of harvested methane is flared and the transformation of methane to liquid chemicals are costly, multi-process reactions. The biggest challenge is preventing over-oxidation, as primary intermediates and products are more reactive than methane and thus preventing high methanol yields.

The most common way for this reaction to proceed is via synthesis gas, which involves high temperatures and pressures and multiple reactions. This is a very costly way to generate methanol and is specifically unfavourable for a small on-site plant. An alternative is the direct, high temperature reaction of methane with oxygen, which is performed under high pressures. An alternative system, well-known in academia, is the oxidation of methane to methyl bisulfate using SO₃ as oxidant (e.g. R. A. Periana et al., Science, 259, 1993, 340 and R. A. Periana et al., Science, 280, 1998, 560). This process is relatively sophisticated, as there is an additional step for the hydrolysis of methyl bisulfate, and the reaction mixture is corrosive. The catalysts employed in this reaction are either mercury-, being environmentally unfavourable, or platinum-based, which is high in costs. In 2005, a step-wise process has been developed in which a copper-loaded zeolite (e.g. ZSM-5 or MOR) is activated in a flow of oxygen (or helium with a subsequent activation step in oxygen) at high temperatures (higher than 280°C, e.g. 450°C)(WO2011046621 A1, M.H. Groothaert et al., JACS, 127, 2005, 1394 and P.J. Smeets et al., Catal. Today, 110, 2005, 303), subsequently oxygen is removed from the reactor and methane is introduced to react with the catalyst (typically between 100°C and 250°C).

Because of the characteristic of the material, methane is oxidized to form a surface-bound methane derivative. In a next step methanol can be extracted from the catalyst by bringing the catalyst into contact with water. This step can either be performed off-line, by removing the catalyst from the reactor and suspending it in a solvent or online by flowing a water-containing stream through the catalyst bed. Typical yields with this method, reported in literature are 13 µmol_{MeOH}/g_{cat} and 5 % methane diluted in either helium or nitrogen is used at ambient pressures.

Recently, a process has been invented, in which copper zeolites and bimetallic copper-noble metal zeolites were used for the conversion of methane (CN101875016 A). The reaction is carried out by employing relatively high pressures (5 - 100 bar) of mixtures of methane and air. Methanol was obtained as sole product (>99.9 %). In such invention, methane is only present in a concentration of 4 % or higher than 50 %. Because the mixing of methane and oxygen may lead to explosive mixtures, there are significant restrictions on the concentrations and ratios of the individual components.

There is currently no precedent or follow-up in the patent and scientific literature showing the 99.9% selectivity at the satisfying conversion levels.

The objective of the present invention is to provide a process for methane to methanol conversion which has high selectivity while providing advantageous and energy-saving conditions for this process.

This objective is achieved according to the present invention by a process for methane to methanol conversion at temperatures below 280°C using oxygen; comprising the steps of:
a) activating a catalyst material with an oxygen-containing gas, preferably molecular oxygen;
b) reacting methane with the activated catalyst material thereby reacting methane to a methanol precursor;
c) reacting and/or desorbing the methanol precursor from the catalyst material by treating with a gas or a liquid such as water, protic molecules, and/or CO;
wherein the steps mentioned above are executed one after the other, thereby applying a temperature in the range of 20 to 280°C, feeding methane and the oxygen-containing gas, at low pressure or at pressures up to 250 bar and using as catalytic material a single- and/or multi- transition metals loaded porous material.

This process allows the conversion of methane to methanol with high methanol yields, advantageous energy consumption for the conversion process without the presence of mixed methane and oxygen containing gas. Major improvement in the present process is the temperature that can be kept significantly below temperatures so far disclosed in the prior art; the whole process can be executed isothermally.

Preferred reaction conditions can be achieved when the temperature is kept constant at a level in the range of 175 to 225°C. Experimental data confirms that a temperature range around 200°C delivers high methanol yields.

In this process, a preferred embodiment of the present invention which delivers high methanol yields provides for catalyst materials that are zeolites, preferably a copper-exchanged mordenite and/or a ZSM-5 and/or a zeolite Y. The catalyst material may comprise an additional transition metal such as platinum.

Preferred reactions conditions with respect to the pressure can be achieved when the pressure for methane and/or the oxygen containing gas ranges from ambient pressure to 250 bar. Preferred examples of the present invention are hereinafter described with reference to the attached figures which depict in:
- Figure 1: schematically illustrates a catalytic cycle for the low temperature reaction from methane to methanol using copper loaded zeolites;
- Figure 2: schematically illustrates the three step process for the conversion of methane to methanol using molecular oxygen;
- Figure 3: the effects of pressure and activation time during the activation step;
- Figure 4: the methanol yield at different methane pressures;

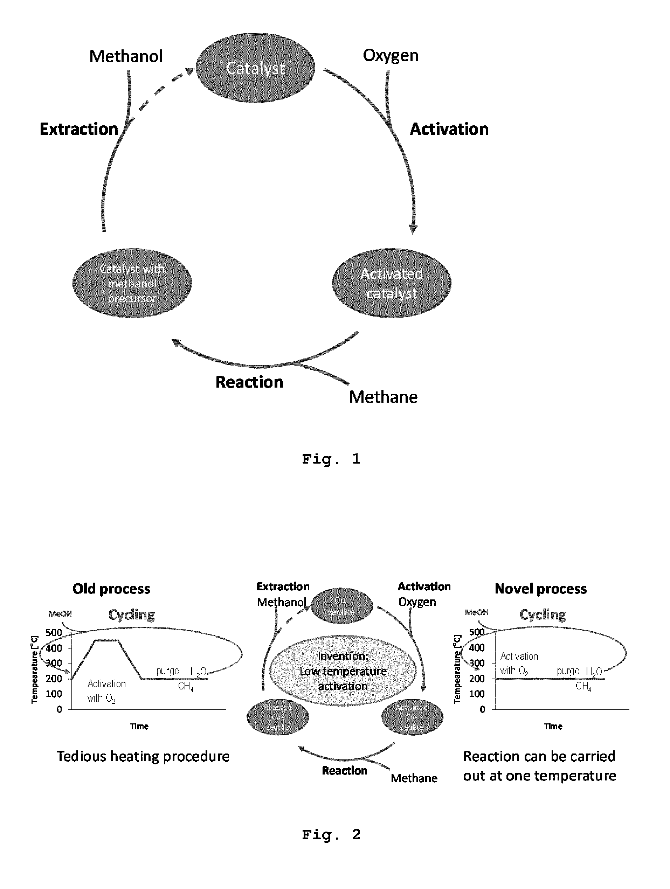

Figure 1 illustrates schematically a catalytic cycle for the low temperature and isothermal reaction from methane to methanol using copper-loaded zeolites according to the present invention. The following represents the state of the art prior to our invention. First, the reactor has to be heated up to the activation temperature in a flow of oxygen, dwell for a certain time, cooled down to reaction temperature, after which the reaction of methane can take place and then the extraction has to be carried out. The present invention provides a way to simplify this process, in which the activation and the reaction can be carried out both at low temperature and even isothermally below 280 C.

The process according to the present invention is substantially different from the processes disclosed in the prior art(CN101875016 A). In the process presented herein, the material is activated, reacts with methane and methanol is extracted. This means that the reagents are introduced separately and are admitted in pure form, in contrast to admission of a mixture of reactants oxygen and methane, which may lead to explosive mixtures, putting limits on the range of possible concentrations.

Avoiding high temperature activation, thus opening the possibility to perform the complete process cycle at low temperature and even isothermally, is now achieved in two ways. The first is an engineering solution, where, by using a higher pressure of methane, the methanol yield can be increased dramatically. Even catalysts with a relatively low yield in the isothermal reaction at low pressures gave a considerably high yield with increased pressures (Table 1, e.g. 5.4 µmol_{MeOH}/g_{cat} at ambient pressures vs. 21.2 µmol_{MeOH}/g_{cat} at ambient pressure of oxygen during activation and 6 bar of methane). It was found that the oxygen pressure has only a minor influence on the yield, whereas an increase in methane pressure results in a greatly increased yield of methanol. The methanol yields are given as µmol·g⁻¹ after off-line extraction with water and analysis via GC/FID. If not stated otherwise, absolute pressures are given as pressures herein.

**Table 1: Results for the isothermal conversion of methane to methanol at 200°C using different pressures. Pressure is given in bar**

| Pressure O₂ | Pressure CH₄ | T (°C) | Methanol yield [µmol·g⁻¹] |
|---|---|---|---|
| 1 bar | 0.05 bar using 5% methane in helium | 200 | 0.3 |
| 1 bar | 1 bar using 5% methane in helium | 200 | 2.2 |
| 1 bar | 1 bar using pure CH₄ | 200 | 5.4 |
| 1 bar | 6 bar using pure | 200 | 21.2 |
| | CH₄ | | |
| 6 bar | 1 bar using pure CH₄ | 200 | 3.5 |
| 6 bar | 6 bar using pure CH₄ | 200 | 16.8 |

The second solution is related to the nature of the catalyst. There are materials which are intrinsically active at low temperature, preferably at 200°C and up to 280°C, even at ambient pressure, without activation at a higher temperature. For example, preparing a copper mordenite with a specific silicon/aluminium ratio, it is possible to increase the yield (Table 2).

**Table 2: Yields obtained with copper mordenite synthesized from NaMOR with different silicon to aluminum ratios. The methanol yields were obtained by activating the catalyst in oxygen at ambient pressure overnight and subsequent methane reaction, all at 200°C.**

| Si/Al molar ratio [-] | Methanol yield [µmol_{MeOH}/g_{cat}] |
|---|---|
| 8.5 | 0.8 |
| 6 | 4.3 |

Additionally, bimetallic catalysts were successfully employed in this process. A significant increase of activity at low temperature was observed by using copper-transition metal, preferentially platinum, mordenite. By investigating different platinum loadings, two 'trends' were shown. During activation with oxygen at high temperature and reaction with methane at 200°C, the methanol yield decreased together with increasing platinum content. At lower activation temperatures, i.e. preferred isothermal conditions at 200°C according to the present invention, the methanol yield increased (Table 3). The best results were achieved by lowering the copper loading in the catalyst and employing a considerably high platinum loading. The maximum yield gained at ambient pressures by this method was 7.4 µmol_{MeOH}/g_{cat}.

**Table 3: Methanol yields using Pt-Cu-mordenite materials with varying Pt concentrations, whereas the last material has a lower copper loading. Yields were obtained at ambient pressures and the complete cyclic reaction was carried out isothermally at 200°C.**

| Expected platinum loading [wt%] | Methanol yield [µmol·g⁻¹] | |
|---|---|---|
| | Activation at 200 °C | Activation at 450 °C |
| 0 | 0.8 | 30.1 |
| 0.2 | 1.2 | 17.7 |
| 1 | 1.7 | 11.2 |
| 2.6 | 3.8 | 4.1 |
| 2.6 (low Cu) | 7.4 | 2.7 |

The extraction can be carried out on-line with steam after reaction and condensing the reactor effluent. The catalyst gives conversion over several cycles.

In summary, two options were analysed in detail, in which methane can directly and selectively be converted to methanol at low temperatures significantly below 280 °C using molecular oxygen as oxidant. This has been done by either employing higher pressures of methane or by designing intrinsically active catalysts that work at low activation temperatures. Indeed, the combination of these two options shows best results.

The cyclic process comprised of three steps according to the present invention is described in detail below. The first step is the oxidation in oxygen-containing gas. After a given period of time, the oxygen-containing gas is removed from the reactor and by introducing methane, the catalyst is partially reduced and activated methane-species are generated on the material. These species are strongly bound and do not desorb during the methane treatment. This is a measure to prevent over-oxidation of carbon-containing species, as methanol is more reactive than methane. Instant desorption would generate free methanol, which would result in over-oxidation and ultimately in total combustion. This leads to low yields of other processes for the direct conversion of methane to methanol. After the interaction with methane, water (or any other reagent or solvent) is introduced as liquid in case of off-line extraction or as water-containing gas stream in case of on-line extraction, which was proven at laboratory scale.

Figure 2 illustrates schematically the three step process for the conversion of methane to methanol using molecular oxygen. Novel is the ability to produce methanol without activation at high temperature, which allows to react methane preferentially at the same temperature or different but lower than 280°C, and therefore all steps can be executed isothermally as shown in the right part of Figure 2, contrarily to the left part in Figure 2 showing the prior art cycle. Until now, there is no report describing a high yield method without the high temperature activation in the cyclic process as shown in the left part of Figure 2. The present invention steps in here. The elimination of the high temperature activation renders the process easier to carry out, without the need of temperature swings -in case of commercial application- in large volumes and reactors. Thus, industrial applicability could be achieved more easily using this method. The present invention is uniquely able to carry out catalyst activation in oxygen at 200°C, subsequently the solid is put in contact with methane and methanol is then released using water and/or protic solvents and/or CO, all preferentially at the same temperature or at temperature lower than 280°C. As described previously this is possible by (i) using higher pressures and/or (ii) using specific catalyst materials.

As mentioned before, the three-step process consists of (i) activation in oxygen-containing gas, (ii) reaction with methane and (iii) treatment with a liquid or a gas (most preferably water) to extract methanol. Two approaches were found that enable lowering the activation temperature and the aforementioned solutions are summarized below. The first solution is based on the pressure enhancement of methane; the second applying a new catalytic material. Without these two new findings, lowering the temperature of activation produces no or very little methanol. The prior art closest to the present invention system is a catalytic (not step-wise) process (CN101875016A) and the step-wise process with high temperature activation (JACS, 127 (2005) 1394-1395, WO2011046621). The first has the problem of possibly generating explosive mixtures and the latter involves complicated heating and cooling procedures.

### Employing higher pressures during the reaction

All prior reports of the closest prior art employ diluted methane gas mixtures (4 - 10 % in either air (catalytic system) or an inert gas (step-wise process). Presently, the effect of pressure has been investigated in the different reaction steps. Copper-exchanged zeolites are preferred, though other catalysts are likely to work. The catalysts were prepared by using the Na-form of zeolites and stirring them in Cu²⁺-acetate solutions. Typically those catalysts have a few wt% of copper. All steps were preferably carried out at the same temperature, although temperatures up to 280°C are possible.

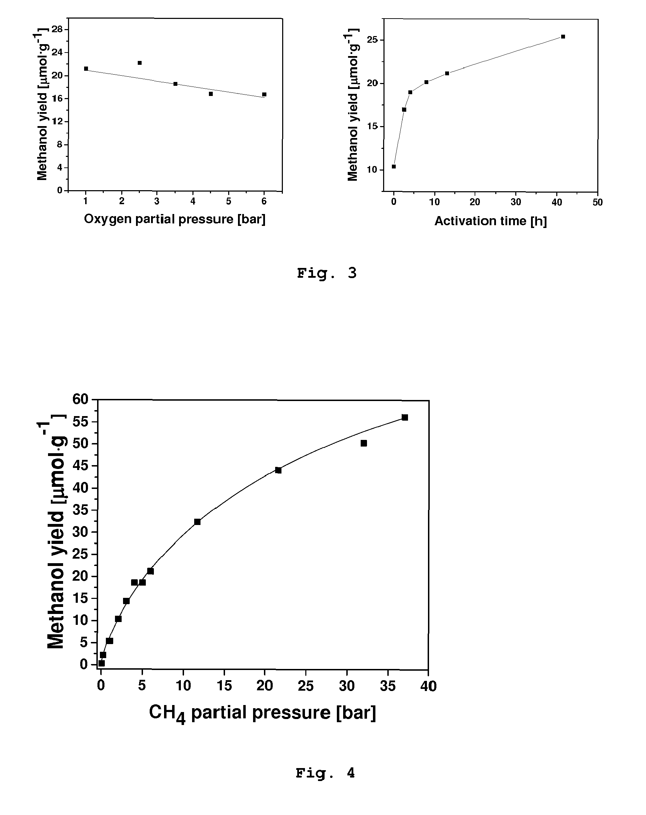

A slightly negative influence of the oxygen pressure during activation has been observed, whereas increasing activation time has a positive effect on the yield (Figure 3). One advantage of the presented step-wise process is that each step can be tuned to increase the overall yield.

Figure 3 shows the effects of pressure and activation time during the activation step. The temperature is not the exclusive parameter. This can be seen by carrying out the reaction using 5 % methane in helium (methane partial pressure = 50 mbar). After high temperature (450°C) activation and reaction at 200°C, 14.4 µmol·g⁻¹ methanol were extracted. Under otherwise identical conditions (i.e. ambient pressures and 13 h activation time), but after activation at 200°C only 0.3 µmol·g⁻¹ have been attained. This proves that not only the temperature, but also the methane pressure is an important parameter. Yields up to 56.2 µmol·g⁻¹ could be achieved at a methane pressure of 37 bar, corresponding to a 187-fold improvement. Generally higher pressures yield more methanol. Presently, a broad pressure range up to 250 bar has been found to deliver superior methanol yields.

Figure 4 shows the methanol yield at different methane pressures. Another advantage of the step-wise process compared to process in which the catalyst is exposed to oxygen after and possibly during methane activation has been found. A reaction, in which the catalyst has been exposed to oxygen-containing gas after reaction with methane (six bar) yielded 5.7 µmol·g⁻¹ instead of 21.2 µmol·g⁻¹, if the catalyst is not exposed to oxygen at 200°C after the reaction. Furthermore, the reaction was proven to work under isothermal conditions between 175°C and 225°C and different zeolites were also shown to yield methanol, i.e. copper-exchanged mordenite (data presented herein), ZSM-5, zeolite Y, and omega.

### Employing different catalysts for the reaction

The second option to attain low temperature activation, thus enabling isothermal process operation, is the usage of specific catalyst materials. In the following section, the reaction has been carried out under isothermal conditions at 200°C and one bar of both oxygen and methane have been used. For copper-exchanged mordenite, it was found that more methanol can be extracted with catalyst prepared in an identical way with lower Si/Al-ratio (Table 2).

Another option is the usage of bimetallic materials. Platinum is an example of such secondary metal. Yields of up to 7.5 µmol·g⁻¹ were achieved by adding platinum to a copper-exchanged mordenite. Different preparation techniques were successful, such as impregnation and ion-exchange of platinum-species. The reference reaction with only Cu-MOR resulted in a low yield of 0.8 µmol·g⁻¹. Other transition metals can also be employed.

## Claims

1. A process for methane to methanol conversion in which each step is executed at a temperature below 280°C; comprising the steps of:
a) activating a catalyst material with an oxygen-containing gas, preferably molecular oxygen, or an inert gas such as helium, nitrogen and argon;
b) reacting methane with the activated catalyst material to form methanol precursors;
c) reacting and/or desorbing the methanol precursor from the catalyst material by treating with a gas or a liquid such as water, protic molecules, and/or CO;
wherein the steps mentioned above are executed in successive order, thereby applying a temperature in the range of 20 to 280°C, providing methane and the oxygen-containing gas at ambient pressure or at a pressure up to 250 bar, and using as catalyst material a transition or multi-metal loaded porous material.

2. The process according to claim 1, wherein the temperature stays constant in the range of 20 to 280°C, preferably in the range of 150°C to 250°C.

3. The process according to claim 1 or 2, wherein as catalyst material a zeolite is used, preferably a copper-exchanged mordenite and/or a ZSM-5 and/or a zeolite Y and/or omega.

4. The process according to claim 3, wherein the catalyst material comprises at least one additional transition metal such as, but not limited to, platinum or other transition metal-species.

5. The process according to any of the preceding claims, wherein the pressure for methane and/or the oxygen containing gas ranges from below 1 bar pressure to 250 bar.
